# EUROPEAN PATENT SPECIFICATION

(11) **EP 4 431 391 B1**
(45) Date of publication and mention of the grant of the patent: **14.01.2026**
(21) Application number: 23162127.7
(22) Date of filing: 15.03.2023
(51) Int. Cl.: B64D 11/02, B64D 11/04, A61L 2/18, C02F 1/68

(54) **WATER SUPPLY AND DISTRIBUTION SYSTEM ON-BOARD AN AIRCRAFT AND METHOD FOR HYGIENIC AND EFFICIENT OPERATION OF SUCH SYSTEM**
WASSERVERSORGUNGS- UND VERTEILUNGSSYSTEM AN BORD EINES FLUGZEUGS UND VERFAHREN ZUM HYGIENISCHEN UND EFFIZIENTEN BETRIEB SOLCH EINES SYSTEMS
SYSTÈME D'ALIMENTATION ET DE DISTRIBUTION D'EAU À BORD D'UN AÉRONEF ET PROCÉDÉ DE FONCTIONNEMENT HYGIÉNIQUE ET EFFICACE D'UN TEL SYSTÈME

(43) Date of publication of application: 18.09.2024
(73) Proprietor: Airbus Operations GmbH, 21129 Hamburg (DE)
(72) Inventor: Albers, Frederik, 21129 Hamburg (DE); Schreiner, Axel, 21129 Hamburg (DE)

(56) References cited:
- EP-A1- 4 080 297
- US-A1- 2018 291 595
- US-A1- 2019 365 936
- US-B2- 10 315 139

## Description

The present invention is directed to a method for operating a water supply and distribution system on-board an aircraft. The invention is further directed to a water supply and distribution system and an aircraft comprising such system.

Conventional water supply and distribution systems on-board commercial aircraft comprise pipework made from rigid pipes, i.e., rigid plumbing. Through the rigid pipes potable water is supplied from a central water tank to consumer assemblies comprising e.g. lavatories having sinks and toilets or galleys with steam ovens and sinks. In such prior art systems in order to convey the water from the central tank to the consumer assemblies, the tank is pressurized by the compressed air, i.e., bleed air or an air compressor generate an overpressure state in the central water tank. Alternatively, centrifugal pumps are employed to convey the water to the consumer assemblies.

US 2018/291595 A1 discloses an aircraft with a drinking water supply and distribution system including a water storage tank, a consumer equipment, a pressurizer, a pressure reducer and a conduit system connecting the drinking water storage tank to the consumer equipment via the pressurizer and supplying water from the at least one water storage tank to the at least one consumer equipment.

EP 4080297 Aldiscloses a water supply system for an aircraft with a plurality of consumers with buffer tanks, which are connected to a water tank via pipes and a pressure source. In each consumer with a buffer tank, a filling valve is arranged between the buffer tank and the pressure source. The buffer tank can be filled by opening the filling valve. By opening a consumer valve, water can be drawn from the buffer tank in each consumer.

Recently, a high pressure water supply system was introduced and it is described in EP 3 385 361 A1. Such industry-optimized water system architecture is based on the concept of a reduction of pipe cross-sections accompanied by an increase in pressure-level as well as a change of pressurization technology, i.e., rather than employing pressurized central water tanks or centrifugal pumps, displacement pumps are used. This adaption allows massive savings, e.g., weight, installation time, equipment cost etc.

Such high pressure water systems can be used in an aircraft to provide potable water in a sufficient amount and quality to all kinds of consumer equipment on-board large passenger aircraft. Potable water may contain microorganisms and pathogenic germs that accumulate and grow on the inner surfaces of the water-carrying elements over time. To guarantee hygienic water supply safety typically in a mobile potable water system, a system disinfection must be performed at regular intervals in such systems in order to suppress the accumulation of microorganisms and the potential formation of a biofilm.

As such, hygienic requirements must be fulfilled to guarantee hygienic water supply safety, and a system disinfection must be performed at regular intervals. For this purpose various methods are available, which are essentially based on a rinsing of the system with special disinfection fluids such as chlorine or chlorine-dioxide o more seldom, by thermal treatment with hot water.

However, these methods have several drawbacks. Firstly, in case of the use of chemical disinfection fluids it is required that these mixtures be provided on the ground so that complex ground service equipment is required. The use of aggressive chemicals may also be required in order to ensure the required level of disinfection, as well as to prevent contamination being introduced from the ground service equipment itself. Alternatively in the case of thermal treatments, external ground service equipment for hot water generation and supply must be provided, which involves additional efforts as well.

In terms of disinfection, it is required that growth of microorganisms and pathogenic germs in the water-carrying elements over time is prevented and those microorganisms and germs are removed. Furthermore, it should be achieved that deposits on the inner walls of water-carrying components such as limescale is fully or at least partially dissolved and that flushing the dissolved particles and other free particles out of the system is carried out. These deposits and particles could reduce the hydraulic cross-section of water pipes, block moving elements such as valves, clog filters and nozzles etc. Especially, regular descaling prevents significant layers of limescale and maintains the functionality of the system.

In particular, manual support of a partially automated process is required or the complete disinfection process has to be performed manually which involves additional work which has to be carried out by additional service personnel which also involves additional costs. In any case the disinfection process is accompanied with an operational interruption with significant and high costly downtime of the aircraft in question.

Thus, it is an object of the present invention to provide a method for operating an on-board water supply system in an aircraft as well as such system which allow for an efficient treatment process without the need for significant maintenance efforts. The treatment process may involve at least one, or both of, a disinfection and a descaling treatment.

The object underlying the present invention is solved by the subject matter defined in the independent claims. Preferred embodiments are described in the dependent claims.

In particular, in a first aspect of the present invention, the above object is solved by a method for operating an on-board water supply and distribution system of an aircraft for supplying water.

The system comprises a central water tank, at least one first consumer assembly, wherein the, or each, first consumer assembly comprises a supply device and a buffer tank with the, or each, first consumer assembly being configured to supply fluid (e.g. water, a treatment solution, or the like) from the buffer tank via the supply device. The buffer tank may also releasably be coupled to the consumer assembly, e.g. formed as a trolley. The, or each, first consumer assembly each comprise a treatment inlet configured to permit the provision of (e.g. receive) a treatment medium and the transfer thereof into the buffer tank. In particular, the buffer tank of the, or each, first consumer assembly each comprise such treatment inlet. It is also conceivable that the treatment inlet is provided in a conduit section connecting the respective buffer tank and the high pressure conduit system.

In particular, in a preferred embodiment, the buffer tanks are configured to hold both water and a treatment medium to provide a treatment solution of a desired concentration to achieve effective treatment of the system. The desired concentration of the treatment solution may be achieved when a treatment medium and water have been added to the buffer tank, to fill the buffer tank to a maximum fill level.

In this context, treatment of the system may involve at least one of disinfection of the system and descaling of the system. Thus, the desired concentration of the treatment solution should be high enough to effectively eliminate or suppress the growth of germ colonies and/or to remove limescale from the surfaces of the system coming into contact with the water. In particular, further growth of microorganisms and pathogenic germs in the water-carrying elements over time is prevented and those microorganisms and germs are removed.

The maximum fill level may correspond to the fluid level of the buffer tank when the buffer tank contains the maximum volume of fluid (e.g. water, treatment medium or solution, or the like) that the buffer tank in question is rated (e.g. intended, configured, permitted) to hold.

Alternatively, the maximum fill level may correspond to a fluid level when the buffer tank contains sufficient water and treatment medium to provide a desired concentration of a treatment solution. The maximum fill level may therefore be determined (e.g. calculated) based on the volume and/or concentration of the treatment medium provided to the buffer tank. Alternatively, the maximum fill level may be another predetermined fill level (e.g. 100%, 95%, 90%, or the like, of the rated capacity of the tank), and the quantity and/or concentration of the treatment medium may be determined (e.g. calculated) based on the known volume of fluid required to provide the maximum fill level.

In one embodiment, the buffer tanks are configured such that they can be filled to a predetermined first fill level before a treatment medium is received therein, and then subsequently filled to a maximum fill level. The first fill level may be, for example, 50% of the maximum fill level of the buffer tank, although there are other possibilities such as 10%, 20%, 25%, 30%, 40%, or the like. In providing a first fill level before providing the treatment medium in the buffer tank, the treatment medium may be distributed more homogeneously throughout the treatment solution, for example because of the mixing effect of inflowing water, and/or because the inflowing water is permitted to mix directly with a treatment medium in liquid form (as opposed to in solid form, or contained in a capsule). Especially in this case, it is also conceivable that the entire volume of water delivered to the buffer tanks is higher than the capacity of the respective buffer tanks so as to achieve that treatment solution flows through overflow lines connected to the buffer tanks and that this part of the system is also treated.

Further, it is to be noted that the first consumer assemblies of the present invention may comprise more than one supply device, e.g., a first consumer assembly in the form of a lavatory comprises a buffer tank provided with a treatment inlet and as supply devices a toilet and a sink with a faucet. In addition, besides first consumer assemblies comprising a buffer tank with a treatment inlet, the aircraft provided with the system of the present invention may comprise additional consumer assemblies that have a buffer tank without a treatment inlet.

Further, the system comprises a pump having an upstream side and a downstream side and being configured such that it may operate in a supply mode and a reverse mode. In a preferred embodiment the pump is configured as a positive displacement pump and further preferred as a gear pump. However, the present invention is not limited to such pumps. It is also conceivable that the supply mode and the reverse mode are achieved by a combination of valves upstream and downstream a pump which taken solely operates in a single mode only, and bypass lines connecting the switching valves. Such combination would also to convey water both from the upstream side to the downstream side (supply mode) and from the downstream side to the upstream side (reverse mode). In such case the upstream and downstream sides are located on the side of the valves remote from the pump.

The system comprises also a high pressure conduit system, wherein the central water tank is connected to the upstream side of the pump, wherein the high pressure conduit system connects the downstream side with the plurality of first consumer assemblies. In addition, the high pressure conduit system is configured such that the pump, when operating in the supply mode, is capable of supplying fluid (e.g. water, a treatment solution, or the like) from the downstream side to the buffer tank of each of the first consumer assemblies, and, when the pump is operating in the reverse mode, it is capable of drawing fluid from the high pressure conduit system towards the upstream side.

Moreover, the method comprises the following steps:
- operating the pump in the supply mode so that water from the central tank is supplied to the, or at least one of, the first consumer assemblies wherein the buffer tank of the at least one first consumer assembly is filled to a predetermined fill level,
- providing a treatment medium to the buffer tank via the treatment inlet, and
- operating the pump in the reverse mode so that the treatment medium in the buffer tank of the at least one of the first consumer assemblies passes through a part of the high pressure conduit system and the upstream side of the pump.

Hence, the system which is operated by the method of the present invention comprises at least one of first consumer assembly supplied with water from a central water tank via a pump when being operated in the supply mode, wherein the supply is preferably carried out with the water being at a pressure of e.g. at least 4 bar (400 kPa), more preferred at least 8 bar (800 kPa) and even more preferred at least 9 bar (900 kPa). The specific pressure depends on the size of the aircraft, the number of consumer assemblies being provided with water from the central water tank, the type of equipment, the diameter of the conduits employed in the system, the storage capacity of the buffer tank and the topology of the entire water supply system.

The water flows from the downstream side of the pump to the buffer tanks of the first consumer assemblies which are provided with a treatment inlet so that a treatment medium may be provided to water in the buffer tanks, thereby providing a treatment solution therein. Furthermore, the pump of the system may also be operated in a so-called reverse mode in which it draws water from the downstream side and, hence, from the high pressure conduit system and pushes it towards the upstream side and to the central tank or a fill/drain coupling as discussed below. So, the method of the present invention makes use of a pump having these two modes of operation.

Moreover, according to the method of present invention the above system is operated such that, e.g. after the normal mode of operation has been stopped manually, or automatically e.g. after a predetermined period of time has elapsed, in a first step the pump is operated in the supply mode so that water from the central tank is supplied or conveyed to the buffer tank of at least one of the first consumer assemblies and the respective buffer tank is filled to the predetermined fill level. However, it is also conceivable that more than one or even each of the buffer tanks of the first consumer assemblies are filled with water from the central tank in this step.

In the following step a treatment medium is provided in the buffer tank via the treatment inlet. The treatment medium may be provided at the treatment inlet, for example, by a user, although it is also conceivable that the treatment medium could be taken, pumped, dispensed, or the like from a store of treatment medium without being directly provided by a user (e.g. the treatment medium could be pumped from a tank or store of treatment medium, optionally activated and/or controlled by a user). The treatment inlet may therefore be a port in the wall of the buffer tank, for example comprising a cover and optionally a latch for permitting the treatment inlet to be closed, opened and secured by a user. Alternatively the treatment inlet may be an opening for or comprising a tubular or pipe for providing a treatment medium, and/or may comprise a valve therein, for example a one-way valve. In another option the treatment inlet may also be provided in a conduit section connecting the respective buffer tank and the high pressure conduit system so that the treatment medium may be transferred to the buffer tank due to the flow of water supplied by the pump via the high pressure conduit system.

It is of course also conceivable that, in embodiments in which more than one consumer assembly having a treatment inlet are provided, a treatment medium may be provided to more than one of the buffer tanks.

In examples in which a treatment medium is provided by a user, confirmation of the provision of said treatment medium into the buffer tank may be provided, for example to a control system. A user may provide such confirmation via a communication interface (e.g. a touchscreen, a keyboard, a button, or the like), which may permit the user to indicate that the treatment medium is presently in the buffer tank.

In some embodiments, the buffer tank or tanks may be provided with level sensors connected to the control unit so that the control unit will be enabled to permit or suggest provision of a treatment medium into the buffer tank only once the respective buffer tank has been filled to the predetermined fill level.

In some embodiments, the buffer tank or buffer tanks of the at least one first consumer assembly may comprise a heater. The heater may be located within and/or external to the buffer tank or tanks. The heater may be configured to heat the water and/or the treatment solution to a desired temperature, such as a process temperature. The process temperature may be a temperature at which the treatment solution is optimally effective. Alternatively, the process temperature may be selected such that a balance is provided between the need to heat the water and/or treatment solution, and the efficacy of the treatment solution at an elevated temperature, for example so as to save energy and expense incurred by heating the water and/or treatment solution, while also ensuring that the treatment medium is sufficiently effective. The process temperature may be dependent on the function of the treatment solution, e.g. the optimal temperature or temperature range in which the treatment solution is effective for a given task, such as disinfection and/or descaling. The process temperature may be, for example, between 15 and 30 degrees Celsius, between 20 and 25 degrees Celsius, up to 25 degrees Celsius, 25 degrees Celsius, or the like. In some examples, the actual temperature to which the water is heated up in the buffer tanks is chosen in accordance with the specific conditions to which the aircraft in question is exposed.

Here, it is to be noted that it is also encompassed by the present invention that the step of providing a treatment medium to the buffer tank of the at least one first consumer assembly may be performed before operating the pump in the supply mode so that water is delivered to the buffer tank after the treatment has been provided to the buffer tank. So the order of these two steps can freely by chosen. Further, it is also conceivable that firstly the pump is operated in the supply mode such that the buffer tank of the at least one consumer assembly is filled to the predetermined level, that the treatment medium is provided to the buffer tank and that then a further amount of water is supplied to the at least one consumer assembly which would result in a better mixing of the treatment medium and the water.

In a following step, the pump is operated in the reverse mode, so that the treatment solution is drawn away from the at least one or more buffer tanks (e.g. some or all of the buffer tanks) of the first consumer assembly or assemblies and passes through a part of the high pressure conduit system, namely that part connecting the respective first consumer assemblies and the downstream side of the pump, from which it is then further conveyed to the upstream side of the pump.

In this following step at least a portion, or all, of the high pressure conduit system is flushed with the treatment solution so as to disinfect and/or descale this portion. Here, when level sensors are provided in the buffer tanks the sensors being connected to the control unit, the reverse mode may be stopped or it is switched to other buffer tanks, when a certain buffer has been emptied.

This method provides for the following advantages. Flushing with treatment solution can be carried out without the need for additional equipment and no ground service involving service personnel is required. The treatment process involving flushing with treatment solution as specified by the method of the present invention can be performed with minimal manual intervention, requiring only of the user the low-skilled task of providing a treatment medium into the system, and with a reduced need to heat water and/or the treatment solution as compared to thermal treatment methods. Hence, the flushing with treatment medium may be carried out while minimising operational interruptions, i.e., it can be carried out on ground and in principle also during flight.

With regard to the step of operating the pump in the reverse mode, it is to be noted that in a preferred embodiment operation of the pump is intermittently stopped for a predetermined period of time so as to achieve that the treatment solution stays especially in the highpressure conduit system for a sufficiently long time to obtain the required efficiency of disinfection.

Moreover, when the above method steps are carried out it is preferred that the consumer assemblies are blocked such that e.g. passengers cannot get in contact with the treatment solution. To this end doors of lavatories may automatically be locked and or the supply devices are prevented from being operated by a user.

In a preferred embodiment, the entire system may be drained after the system has been flushed with the treatment solution. Draining may be achieved by pumping the treatment solution from the central tank into a drain (located inside or outside the aircraft, in cases in which the system is treated on the ground) or into a waste fluid container. It is also possible that draining the central tank is achieved by gravity so that the treatment solution reaches the drain without using a pump.Should any fluid and/or treatment solution remain in the buffer tanks, the fluid and/or treatment solution may be pumped to the central tank, via the process described above, before draining. After draining, the entire system may then be flushed with fresh water. Fresh water may be provided to the central tank from, for example, an external source or a further fresh water tank. Once in the central tank, the pump may be used to pump fluid into the or each buffer tank, before reverse pumping the fluid back to the central tank and draining once more. Alternatively, the fluid used for flushing may be supplied to the consumer assemblies. This step may be performed automatically, for example once the control unit detects that the central tank has reached a maximum capacity of treatment solution and/or once all the fluid has been pumped from the buffer tanks to the central tank. The system may therefore prepare itself for further use by users. In this regard, it must be ensured that all parts of the system which have been in contact with the treatment solution are also rinsed, i.e., when overflow line connected to the buffer tanks was in contact with the treatment solution, it must also be rinsed.

Furthermore, in a preferred embodiment the supply device of each of the at least one first consumer assemblies is operated such that treatment solution is also supplied via the respective supply device, i.e., the treatment solution is not only conveyed through the high pressure conduit system by means of the central pump but also guided through the conduits to the respective supply device so as to achieve disinfection of the respective portions of the entire water supply system. For example, treatment solution may be supplied to a toilet which results in collection the treatment solution into the waste holding tank of the aircraft's toilet system. It may also be supplied via a faucet into the wash basin which results in the following two options. It is further conveyed to the drain system, i.e. draining the fluid to the overboard via the drainmasts, or to the collection system, i.e., the treatment solution is collected into the waste holding tank.

In a preferred embodiment, the system comprises at least one, or a plurality of, first consumer assembly, and the, or each, of the at least one or plurality of first consumer assemblies are provided with an inlet valve, which is arranged between the buffer tank and the high pressure conduit system, each of the inlet valves having a closed position and an open position, wherein in the step of operating the pump in the supply mode, water from the central tank is supplied to the at least one, or plurality of, the first consumer assemblies wherein the buffer tank or tanks of the plurality of the first consumer assemblies are filled to a predetermined fill level, wherein the treatment medium is provided to the buffer tank of the at least one or plurality of first consumer assemblies via the respective treatment inlet to mix with the water in the buffer tank or tanks to form a treatment solution, and wherein in the step of operating the pump in the reverse mode, the inlet valves of the at least one or plurality of first consumer assemblies are controlled to be in the open position so that the treatment solution in the buffer tank or tanks of the at least one or plurality of first consumer assemblies passes through a part of the high pressure conduit system and the upstream side of the pump. Here, it is preferred, when the filling level in the respective buffer tank is monitored, e.g. via a level sensor, so that the inlet valve will be closed when the buffer tank has been emptied or reached a minimum filling level to avoid that air is drawn into the high pressure conduit system.

Furthermore, when the system comprises a plurality of first consumer assemblies each being provided with an inlet valve, it is preferred that during the step where the pump is operated in the reverse mode so as to draw the treatment solution through the high pressure conduit system, the inlet valves are opened and closed in a predetermined order so that at any point in time only one or a group of inlet valves is open whereas others are closed and not all of the buffer tanks of the first consumer assemblies are emptied at the same time, but the buffer tanks are subsequently emptied. This ensures that a controlled flow of the treatment solution through the high pressure conduit system is achieved.

In a further preferred embodiment, the at least one or plurality of first consumer assemblies are filled to an initial predetermined fill level, wherein the treatment medium is provided to the buffer tank of the at least one or plurality of first consumer assemblies via the respective treatment inlet to mix with the water in the buffer tank or tanks to form a treatment solution, and wherein the pump is subsequently further operated in the supply mode to fill the buffer tank or tanks to a maximum fill level, and wherein in the step of operating the pump in the reverse mode, the inlet valves of the at least one or plurality of first consumer assemblies are controlled to be in the open position so that the treatment solution in the buffer tank or tanks of the at least one or plurality of first consumer assemblies passes through a part of the high pressure conduit system and the upstream side of the pump. In such a preferred embodiment, level sensors may be used to facilitate identification of the initial predetermined fill level, and the maximum fill level, for example by a control unit.

In a another preferred embodiment of the method of the present invention the connection between the central water tank and the upstream side of the pump comprises a fill/drain coupling and when operating the pump in the reverse mode, so that the treatment solution in the buffer tank of the at least one of the first consumer assemblies passes through a part of the high pressure conduit system and the upstream side of the pump, the treatment solution is passed through the fill/drain coupling and/or to the central water tank. In the first alternative of this preferred embodiment the treatment solution which has been drawn through at least a part of the high pressure conduit system and the pump is either discharged through the fill/drain coupling and does not reach the central water tank, so that there is no risk that the central water tank, and any water therein, is contaminated by treatment solution, should the introduction of treatment solution in the central tank not be desired. In the second alternative, the treatment solution is collected in the central water tank so that it may be disposed of at a later point in time.

In another preferred embodiment of the method of the present invention the system further comprises second consumer assemblies, each second consumer assembly comprising a supply device and a buffer tank and each second consumer assembly being configured to supply water and/or treatment solution from the buffer tank via the supply device, wherein the high pressure conduit system connects the downstream side of the pump with the plurality of second consumer assemblies, with the conduit system being configured such that the pump, when operating in the supply mode, is capable of supplying water and/or treatment solution from the downstream side to the second consumer assemblies, so that the water is received in the buffer tank of at least one of the second consumer assemblies,
- wherein when the pump is operated in the reverse mode so that the treatment solution in the buffer tank of the at least one of the first consumer assemblies passes through a part of the high pressure conduit system and the upstream side of the pump, the treatment solution is passed to the central water tank, and
- wherein the method comprises the following further step of operating the pump in the supply mode so that treatment solution from the central tank is supplied to at least one of the second consumer assemblies, the heated water being received in the buffer tank of at least one of the second consumer assemblies.

The above process may then be repeated, but by operating the pump in the reverse mode so that the treatment solution in the buffer tank of at least one of the second consumer assemblies passes back into the central tank, via the high pressure conduit system, and then operating the pump in the supply mode so that the treatment solution from the central tank is supply to at least one further second consumer assembly (i.e. from at least one initial second consumer assembly, to at least one subsequent second consumer assembly).

In this preferred embodiment it is made use of the fact that the second consumer assemblies are provided with a buffer tank even if the buffer tank does not comprise a treatment inlet. This allows the supply of treatment solution to the second consumer assemblies which was previously stored in the central water tank, so that those parts of the high pressure conduit system connecting the second consumer assemblies and the central can be treated as well. This therefore reduces the manual workload by permitting the system to function with only one, or some, of the available buffer tanks having treatment inlets, and/or by permitting a user to have to provide only one, or some, of the available buffer tanks with a treatment medium.

Here, it is a further advantage that the treatment solution supplied to the second consumer assemblies is then again drawn through the high-pressure conduit system which further improves the efficiency of the treatment process.

Additionally, or as an alternative, the treatment solution in the buffer tanks of the second consumer assemblies may also be conveyed to the supply devices of the assemblies so as to treat, i.e., disinfect and descale, the conduit sections between the buffer tank and the respective supply device which would otherwise not be possible.

Finally, it is preferred that before the pump is operated in the reverse mode and the treatment solution in the buffer tank of the at least one of the first consumer assemblies is passed to the central water tank, the central tank is drained. This may prevent unintended dilution of the treatment medium.

In another aspect of the present invention the above object is solved by an on-board water supply and distribution system of an aircraft for supplying water, the system comprising a central water tank, at least one first consumer assembly, each of the at least one first consumer assemblies comprising a supply device and a buffer tank and each of the at least one first consumer assemblies being configured to supply water from the buffer tank via the supply device, wherein each of the buffer tanks of the at least one first consumer assemblies comprises a treatment inlet for receiving a treatment medium into the respective buffer tank, a pump having an upstream side and a downstream side and being configured such that it may operate in a supply mode and a reverse mode, a high pressure conduit system, and a control unit, wherein the central water tank is connected to the upstream side of the pump, wherein the high pressure conduit system connects the downstream side with the plurality of first consumer assemblies, with the conduit system being configured such that the pump, when operating in the supply mode, is capable of supplying water from the downstream side to the buffer tank of each of the first consumer assemblies, wherein, when the pump is operating in the reverse mode, it is capable of drawing water from the high pressure conduit system towards the upstream side, the control unit being connected to the pump and each of the at least one first consumer assemblies and being configured such that in a first step the control unit operates the pump in the supply mode so that water from the central tank is supplied to at least one of the at least one first consumer assemblies wherein the buffer tank of the at least one of the at least one first consumer assemblies is filled to a predetermined fill level, in a second step a treatment medium is provided to the buffer tank of the at least one of the first consumer assemblies, and in a third step the control unit operates the pump in the reverse mode so the treatment solution in the buffer tank of the at least one of the first consumer assemblies passes through a part of the high pressure conduit system and the upstream side of the pump.

Hence, the system of the present invention is also configured such that it can firstly be operated so that the or each buffer tank of the at least one first consumer assembly is supplied with water from the central water tank, a treatment medium is then provided to the water in the or each buffer tank to provide a treatment solution and finally, when the pump is operated in the reverse mode, the treatment solution is drawn through the high pressure conduit system, so that the latter is treated (e.g. disinfected and/or descaled). Thus, the system of the present invention provides for the same advantages as the above-described method. The same applies to the preferred embodiments of the system as specified in the dependent claims of the respective independent claims describing the system of the present invention.

Finally, the above object is also solved by an aircraft comprising a system as described before.

In the following, the invention will be described further with regard to the exemplary embodiment shown in the drawings, wherein
- Figure 1: shows a schematic drawing of an exemplary embodiment of an on-board water supply and distribution system of an aircraft,
- Figure 2: shows an exemplary embodiment of an aircraft having on-board water supply and distribution system of Figure 1,
- Figure 3: a schematic drawing of a second consumer assembly of the system of figure 1, and
- Figure 4: a flow chart depicting an exemplary embodiment of a method of the present invention.

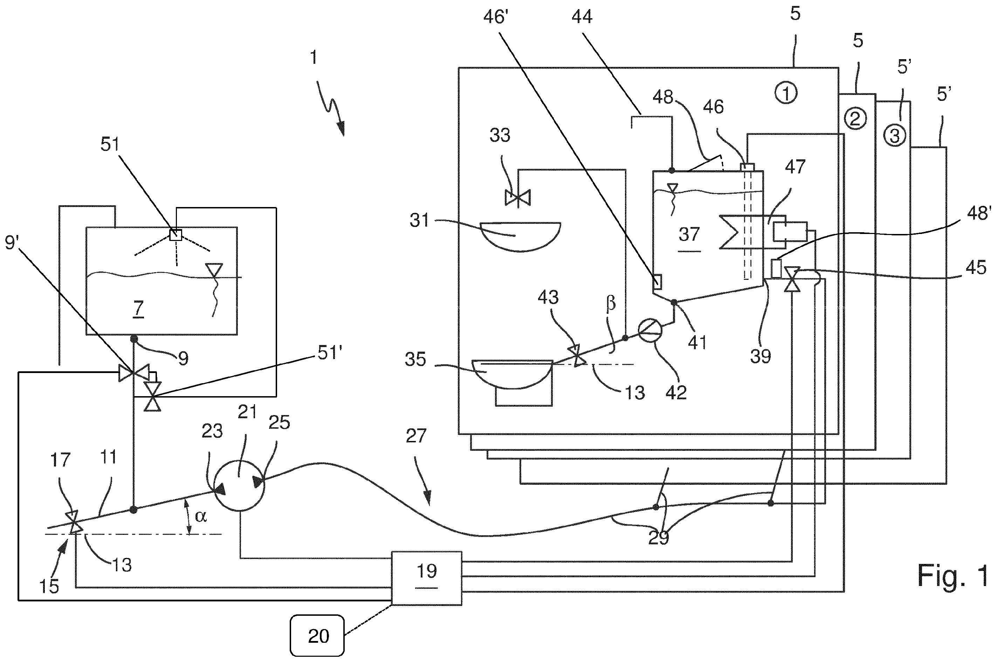

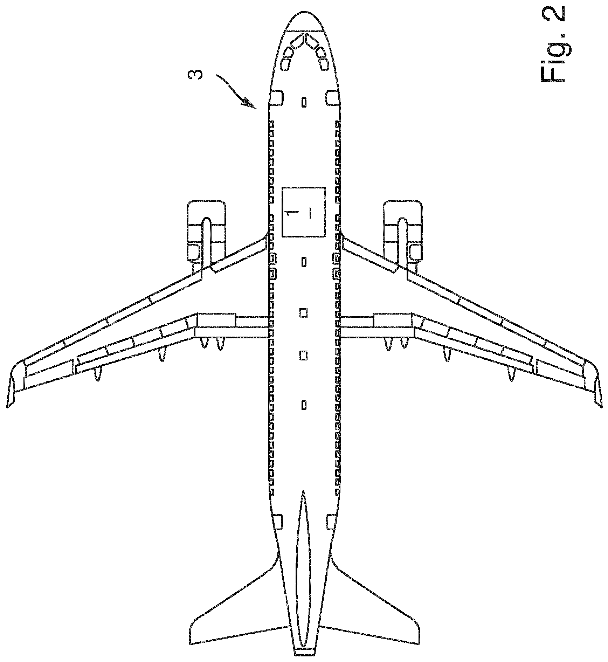

As can be taken from figures 1 and 2 the exemplary embodiment of a water supply and distribution system 1 is generally arranged on-board an aircraft 3 and configured such that it supplies a plurality of first consumer assemblies 5 comprising sinks and toilets in a lavatory or steam ovens and sinks in a galley, with potable water as will be described in detail below. Figure 2 only schematically shows the arrangement of the system 1 in the aircraft 3, and several types of arrangements are conceivable.

The water supply and distribution system 1 comprises a central water tank 7 which is provided with a connector 9 at its bottom with the connector 9 being connected to a supply line 11 which is arranged in this exemplary embodiment in such a manner in the aircraft 3 that it includes an angle α with the horizontal 13 when the aircraft 3 is on the ground and in a horizontal position A controllable valve 9' is provided in the connection between the connector 9 and the supply line 11. Generally, the supply line 11 is inclined downwards to a fill/drain coupling 15 which is the provided with a valve 17 and which is arranged at the free end of the supply line 11. The valve 17 is connected to a control unit 19 of the system 1 and can remotely be controlled such that it can be switched between a closed and an open position. The control unit 19 may be in the form of a programmable unit so that a software may be uploaded to the control unit 19 which configures the unit 19 so that it may operate the system 1 in the manner as described below. However, it is also conceivable that the control unit 19 is in the form of a programmable computer appropriately connected with the components which need to be controlled so as to perform the method as described below. The control unit 19 is connected to a communicationdevice 20 by wire or via a wireless connection so that the system 1 may be controlled by a user via the communication device 20. The communication device 20 may be a control panel provided in the cabin of the aircraft 3.

As can further be taken from figure 1, the supply line 11 connects the fill/drain coupling 15 with a central pump 21 which is also operatively coupled with the control unit 19 so that the control unit 19 may control the mode of operation of the central pump 21. Preferably, the central pump 21 is configured as a positive displacement pump and further preferred as a gear pump. In particular, the central pump 21 can be operated in a supply mode where it conveys water from its upstream side 23 and the supply line 11 to its downstream side 25 that is connected to a high pressure conduit system 27 which will be described in detail below. The high pressure conduit system 27 comprises a plurality of conduits 29 connecting the downstream side 25 of the central pump 21 with the first consumer assemblies 5. Moreover, the central pump 21 may also be operated by the control unit 19 in a so-called reverse mode, in which it draws water from its downstream side 25 and the high pressure conduit system 27 and conveys it to its upstream side 23 and towards the supply line 11.

The first consumer assembly 5 shown in figure 1 is configured as a lavatory with a sink 31 provided with a faucet 33 and a toilet 35 as supply devices. Here, it is to be noted that other forms of first consumer assemblies are conceivable such as galleys having sinks etc., and that the present invention is not limited to consumer assemblies 5 in the form of lavatories.

Furthermore, the first consumer assembly 5 comprises a buffer tank 37 having an inlet 39 and an outlet 41, the latter being connected to the faucet 33 and the toilet 35 via a micro pump 42. As can further be taken from figure 1 the line connecting in the outlet 41 with the toilet 35 is also inclined downwards and in this exemplary embodiment includes an angle β with the horizontal 13, when the aircraft 3 is horizontally arranged on the ground. In general, the outlet 41 is arranged at a higher level than the toilet 35. This ensures that when the valve 43 of the toilet 35 is open, the buffer tank 37 may be completely drained via the toilet 35. However, it is also conceivable that when such configuration with a supply device at a lower level than the outlet of the buffer tank 37 cannot be achieved, a pneumatic drainage procedure is conducted, to empty the buffer tank. Alternatively, it is conceivable that the buffer is arranged below the supply device so that an angle is realized in the opposite direction and water will flow back into the buffer via gravity.

Furthermore, as can also be taken from figure 1, the buffer tank 37 is provided with an overflow line 44 so that when the volume of water supplied to the buffer tank 37 exceeds its capacity, the excess water may be transported to either the toilet 35 or to the wastewater system in general.

In addition, the inlet 39 of the buffer tank 37 of each of the first consumer assemblies 5 is provided with an inlet valve 45 which can be switched between an open and a closed position and which is operatively coupled with the control unit 19, so that the control unit 19 may change the position of the inlet valves 45. The inlet 39 is connected to the downstream side of the central pump 21 by the high pressure conduit system 27 including the conduits 29. It is preferred that when the aircraft 3 is on the ground, the inlet 39 is arranged at a distance in the vertical direction from the bottom of the buffer tank 37 with the outlet 41. This arrangement of the inlet 39 has the effect that when water is drawn out of the buffer tank 37 via the inlet 39 by means of the central pump 21 when being operated in the reverse mode, the buffer tank 37 cannot entirely be emptied and particles which have sedimented at the bottom of the buffer tank 37 are not drawn out of the tank 37 and conveyed towards the pump 21. Instead, these particles can only be removed from the buffer tank 37 via the outlet 41.

Moreover, the buffer tanks 37 are provided with level sensors 46 which are connected with the control unit 19 and are configured to monitor the water level in the buffer tank 37 and to provide a corresponding signal for the control unit 19, the signal being indicative for the height of the level in the buffer tank 37. In addition, the buffer tanks 37 are provided with temperature sensors 46' connected to the control unit 19.

Each of the buffer tanks 37 of the first consumer assemblies 5 are provided with heaters 47 which are connected to the control unit 19 and configured such that when being activated by the control unit 19 they are configured to heat up water within the buffer tank 37 up to a predetermined process temperature, which in this preferred embodiment is 25°C. However, it is within the scope of the present invention that other temperatures may be chosen as predetermined process temperature, such as 35°C or 45°C. The heating up may be monitored by the temperature sensors 46'.

Each of the buffer tanks 37 additionally comprises a treatment inlet 48 for permitting the provision of a treatment medium into the buffer tank 37. Although not illustrated, in the case where there is more than one first consumer assembly 5, each of the buffer tanks 37 comprises a buffer tank 37 having a treatment inlet 48. In this example, the treatment inlet is schematically illustrated, and for clarity is illustrated as a hatch (e.g. a cover configured to be open and shut, e.g. via a hinge joint, a magnetic fastener permitting removal of the cover, or the like) positioned on a top surface of the buffer tank 37 of the illustrated first assembly. However, it should be noted that this illustration is not intended to be restrictive of either the form or the position of the treatment inlet 48 relative to the buffer tank 37. For example, the treatment inlet 48 may be or comprise a hatch located on the side of the respective buffer tank 37, for example on the side of the buffer tank 37 above a maximum fill level of the buffer tank 37. Where the treatment inlet 48 is or comprises a cover, a user may be able to open or remove the cover, thereby providing direct access to the buffer tank 37 (e.g. access to the internal volume of the buffer tank 37), so as to allow a user to provide a treatment medium (e.g. a disinfection and/or descaling medium) to the buffer tank 37.

The treatment inlet 48 may additionally or alternatively comprise an opening (e.g. an aperture) that is located in the buffer tank 37 that is fluidly connected via a conduit (e.g. a tubular, pipe, or the like) to an access opening, accessible by a user. The access opening permits a user to provide a treatment medium to the buffer tank 37 in any appropriate way, such as via a canula, a valve, a diaphragm, a septum valve, a disconnected hydraulic interface such as an overflow line or the like.

As also indicated in figure 1, a treatment inlet 48' may additionally or as an alternative be arranged in a conduit section connecting the inlet 39 of the buffer tank 37 with the high pressure conduit system 27 so that a treatment medium is transferred to the buffer tank 37 when water is supplied to it from the high pressure conduit system 27.

The treatment medium may be in any appropriate form. For example, the treatment medium may be in liquid or solid form. The treatment medium may be in the form of a tablet, e.g. a water soluble tablet, a disinfection and/or descaling fluid, a gel, a powder, or the like. The treatment medium may be contained in a cartridge or cartouche. The cartridge or cartouche may be connectable to a treatment inlet interface (not illustrated) such as a canula or valve, or may be water soluble such that it can be placed by a user in the buffer tank 37, e.g. directly in the buffer tank or transported via a conduit, chute, or the like, into the buffer tank 37.

The treatment inlets 48, 48' may be securable in a closed position, for example to prevent unauthorised use thereof. The treatment inlets 48, 48' may therefore comprise a latch or locking mechanism that is operable only by authorised users, such as via an input code, a key or the like. In some embodiments, the treatment inlet 48, 48' may be securable in a closed position by a screw, a bolt, or the like, that requires specialist equipment to remove such as a screwdriver or bolt removal tool, that an unauthorised person would be unlikely to possess, or that it would not be permissible for an unauthorised person to possess, for example on an aircraft. In addition, the treatment inlets 48, 48' are preferably configured such that when being in the closed position they are water proof and pressure tight.

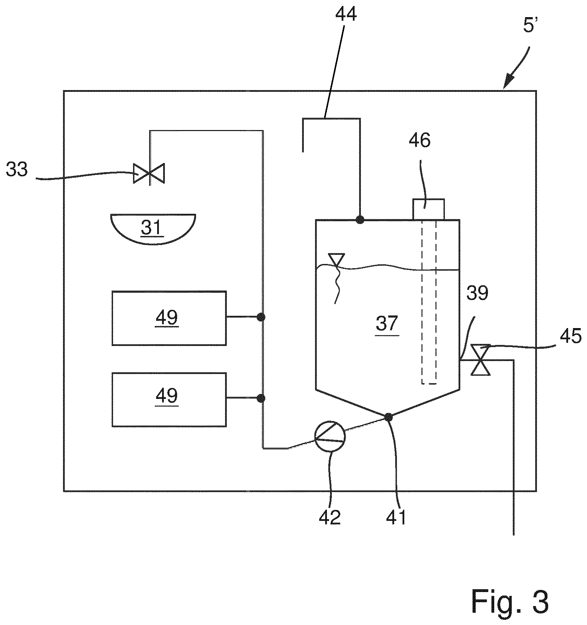

Furthermore, it can also be taken from figure 1 that the water supply and distribution system 1 comprises a plurality of second consumer assemblies 5' which are shown schematically in figure 3 and which differ from the first consumer assemblies 5 merely in that the buffer tank 37 is not provided with a treatment inlet 48, 48', so that a user is not able to provide a treatment medium to buffer tank 37 of these consumer assemblies 5'. However, except for this difference the second consumer assemblies 5' do not differ from the first consumer assemblies 5 as described above, i.e., they may be in the form of a galley being provided with a sink 31 and a faucet 33 as well as further devices 49 for producing beverages other configurations are conceivable and within the scope of the present invention.

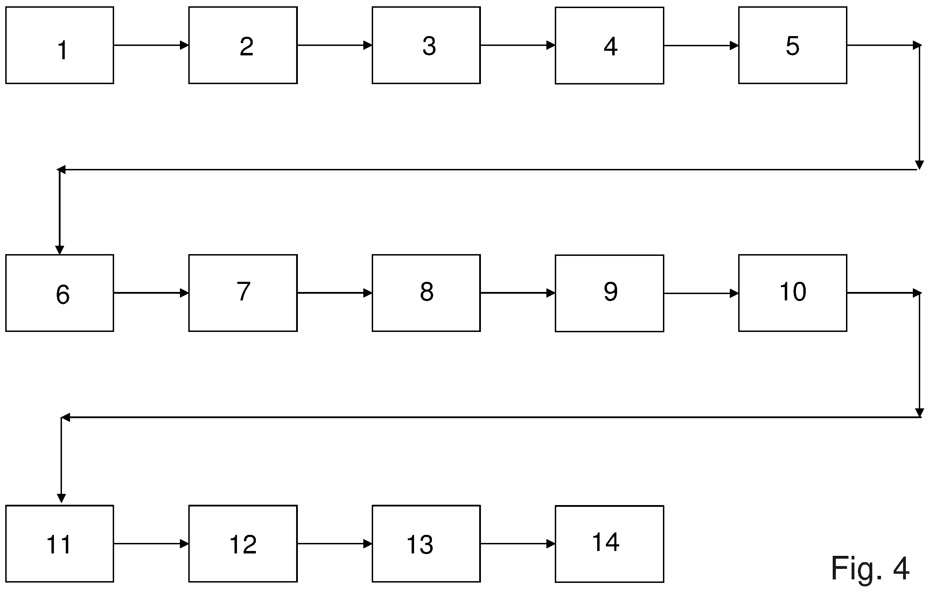

In the following with reference to figure 4 showing a flow of an exemplary embodiment of a method of the invention it will be described how the aforementioned water supply and distribution system 1 of an aircraft 3 is controlled by the control unit 19 so as to perform the inventive treatment process. In figure 4 the different steps of this embodiment are depicted.

After the normal mode of operation in which the first and second consumer assemblies 5, 5' are supplied with potable water from the central water tank 7 by the central pump 21 operating in the supply mode was stopped manually (Step 1) , e.g., by means of the communication device 20, or automatically after a predetermined period of time interval has elapsed, the following first step will be initiated by the control unit 19.

Firstly, the supply devices such as faucets 33 and toilets 35 are deactivated so as to prevent the use thereof by passengers (Step 2). In next step (Step 3) the control unit 19 operates the pump 21 again in the supply mode and the inlet valves 45 of the first consumer assemblies 5 are controlled such so that water from the central tank 7 is supplied to the, or each, of the first consumer assemblies 5 wherein the buffer tank 37 of the first consumer assemblies 5 is filled to a predetermined initial fill level. The predetermined fill level may be, for example, 50% of the maximum fill level of the tank. The maximum fill level may correspond to the maximum volume of fluid that the buffer tank 37 is rated to hold, or may correspond to the maximum volume of a treatment solution that is able to be provided by mixing the treatment medium with water to provide treatment solution of a sufficient concentration to provide the desired treatment (e.g. disinfection, descaling or both).

In the next step (Step 4) the control unit 19 further operates the heaters 47 of the buffer tanks 37 of the plurality of first consumer assemblies 5 in such a manner that the water in these buffer tanks 37 is heated to the predetermined process temperature e.g. of 25°C. However, the control unit 19 activates only the heaters 47 of those buffer tanks 37 where the level sensor 46 provides a signal to the control unit 19 that indicates that a sufficient amount of water is in the buffer tank 37, such that the predetermined initial fill level has been reached.

In a fifth step (Step 5), a treatment medium is introduced into the buffer tank 37 and mixed with the water therein to provide a treatment solution. Although the term "treatment solution" is used here, it should be understood that this refers to the fluid mixture of water and the treatment solution, and which may also take other forms, such as an emulsion. As stated before, the treatment solution may be directly provided into the buffer tank 37 by a user, or may be transported to the tank 37, for example via a conduit. In this step, the user may receive a notification, generated by the control unit 19, and relayed to a user via a user notification such as the communication device 20, a screen, LED, buzzer, or the like. Hence, the user may monitor the entire treatment process.

A sixth step (Step 6) involves the control unit 19 operating the pump 21 once more to provide potable water to the buffer tank or tanks 37 of the first consumer assemblies 5 so as to fill the buffer tank or tanks 37 to the maximum fill level or even beyond that level, so as to achieve that treatment solution flows through the overflow line 42. This step may be triggered by an input to the control unit 19 e.g. via the communication device 20 that the treatment medium has been provided to the buffer tank or tanks 37. The input may be in the form of a confirmation from a user that the treatment medium has been provided to the tanks, although it should be understood that other forms of confirmation are possible, for example from a sensor that is able to detect the treatment inlet 48 being used (e.g. opened or closed, or that a treatment medium is present in a conduit leading to the buffer tank 37) or detect e.g. a change in the pH of the fluid in the buffer tank 37, thereby indicating the presence of a treatment medium in the buffer tank or tanks 37.

Once the treatment medium has been provided to the buffer tank or tanks 37, the control unit 19 in this preferred embodiment is then configured to operate the heaters 47 of the buffer tanks 37 to heat the fluid therein, in this case the treatment solution, to a process temperature e.g. of 25°C (Step 7). Once the treatment solution has reached the process temperature, the control system 19 is configured to allow the treatment solution to remain in the buffer tank or tanks 37 for a predetermined reaction time, to allow the tank to be treated (Step 8).

After the reaction time has elapsed, the control system 19 is then configured to activate consumer equipment, illustrated in Figure 1 as a sink 31, a faucet 33, a toilet 35 or a galley device 49, to provide treatment of said consumer equipment, such as disinfection or descaling (Step 9).

In subsequent Step 10 the control unit 19 operates the pump 21 in the reverse mode, the control unit 19 controls the inlet valve 45 of the at least one, or the plurality of, first consumer assemblies 5, such that it is in the open position. Once the inlet valve 45 the at least one, or the plurality of, first consumer assemblies 5 has (subsequently) been opened by the control unit 19, treatment solution from buffer tank 37 is drawn through at least a part, or all, of the high pressure conduit system 27 to the downstream side 25 of the pump 21 and passed to the central water tank 7 with the valve 17 of the fill/drain coupling 15 being closed by the control unit 19. So, the treatment solution from the buffer tank or tanks 37 is conveyed to the central water tank 7 and is stored therein.

In order to facilitate this, the control unit 19 has controlled the valve 17 of the fill/drain coupling 15 before the third step so as to drain the central water tank 7 before the treatment solution from the buffer tank or tanks 37 have been conveyed to it by the pump 21 operating in the reverse mode. Once the treatment solution has reached the central tank 7, the control system 19 ceases pumping for at least a reaction time period, during which time the treatment solution stays in the central tank 7 (Step 11).

In the case where there is a plurality of first consumer assemblies 5, the control unit 19 may configure the inlet valve 45 to the open position of all of the respective buffer tanks simultaneously, such that the treatment solution of all of the respective buffer tanks 37 is drawn simultaneously through the high pressure conduit system 27 and to the central tank 7. Alternatively, the treatment solution may be drawn from the buffer tanks 37 in a stepwise manner, such that the treatment solution is drawn from the tanks individually, and the inlet valve 45 of a subsequent tank is not configured to the open position until all of the treatment solution from a previous tank 37 has been drawn through the high pressure conduit system 27 to the central tank 7.

Reverse pumping is continued in Step 10 until each of the buffer tanks 37 containing treatment solution have been emptied sufficient treatment solution has been pumped to the central water tank 7 required to treat those parts of the conduit system 27 not being connected (e.g. directly connected) with a first consumer assembly 5. So, once Step 10 has been completed, those parts of the high pressure conduit system 27 interconnecting the downstream side of the pump 21 and the first consumer assemblies 5 have been flushed with treatment solution. In this regard it is to be noted that in this embodiment the concentration and temperature of the water in the buffer tanks 37 is chosen such that sufficient treatment is achieved. In this context, treatment may be that the components of the water supply and distribution system, through which potable water is conveyed, are disinfected and/or descaled so that pathogenic germs and/or limescale on the inner surfaces of the water-bearing parts such as the high pressure conduit system 27 are eliminated or suppressed by said treatment. Further, the temperature is maintained in the high pressure conduit system 27 at or sufficiently close to the process temperature for a reaction time. This facilitates the suppression or prevention of the formation of hygienically relevant germ colonization (e.g. in a biofilm) on the inner surfaces of the high pressure conduit system 27, as well as the formation of limescale.

After a reaction time has elapsed where treatment solution is in the central tank 7 (Step 11), in a further step (not shown in figure 4), the control unit 19 again operates the pump 21 in the supply mode, and the treatment solution from the central tank 7 is pumped through the high pressure conduit system 27 to the second consumer assemblies 5' not being provided with a treatment inlet so that the treatment solution will be received in the buffer tanks 37 of each of the second consumer assemblies 5'. It should be noted that, while in this embodiment there exist a plurality of second consumer assemblies 5', in some other embodiments functioning similarly as has been described above, there may be only one second consumer assembly 5', or there may be no second consumer assemblies 5'.

After the buffer tanks 37 of the second consumer assemblies 5' have been filled with treatment solution from the central tank 7 and treatment solution has been supplied to the supply devices of the at least one second consumer assembly 5', the central pump 21 is again switched to the reverse mode by the control unit 19, and the treatment solution in the buffer tanks 37 of the second consumer assemblies 5' is subsequently (e.g. simultaneously, or in a stepwise manner) drawn back from each of the buffer tanks 37 to the pump 21 and pumped out of the fill/drain coupling 15 with the valve 17 being opened by the control unit 19.

In some embodiments, the treatment solution may be pumped between the buffer tanks 37 of the first and/or second consumer assemblies 5, 5' several times by repeating the steps as outlined above. In doing so the conduit system 27, buffer tanks 37 and central tank 7 may be more thoroughly treated. In particular, this may combat the phenomenon of a lower concentration of treatment solution reaching crevices and narrow gaps between items of equipment.

In a further embodiment, the method may involve pumping fluid into the central tank 7, such that the fluid is sprayed onto an inner surface of the central tank 7 by means of a nozzle assembly . In such an embodiment, the system may be substantially as previously described, with the exception that the central tank 7 comprises a fluid disperser 51, such as a spray nozzle , at or connected to an inlet thereof. In this embodiment, with the pump 21 operating in the reverse mode, the treatment solution is passed into the central tank 7 via a controllable valve 51' connected to the control unit 19 and the fluid disperser 51 such that it is sprayed onto the interior walls of the central tank 7, utilising the pressure provided by the pump 21 in the reverse mode to provide a spray of treatment fluid in the central tank 7. To facilitate the spray coating the walls of the tank, the fluid disperser may be located on an upper or top wall of the central tank 7.

Once a desired volume of treatment solution has been sprayed onto the interior walls of the central tank 7, the treatment solution may be drained from the tank and passed, for example, back into a buffer tank 37 as has been previously described. The control unit 19 may be configured to operate the pump 21 in the reverse mode until a sufficient volume of treatment solution has been sprayed into the central tank 7. A level sensor in the central tank 7 may alert the control unit 19 to the fact that a sufficient volume of treatment solution has been sprayed into the central tank 7. The valve 51' acts as a shutoff valve to facilitate shutoff of the spraying of treatment fluid inside the central tank 7 when required.

This embodiment has the advantage that the phenomenon whereby there is a reduction in the concentration of the treatment solution in the surface layer adjacent the walls of the tank does not occur, as may be the case were the tank to be filled with treatment solution. Further, the volume of treatment solution that is required is significantly reduced compared to examples in which the central tank 7 should be completely filled with treatment solution.

Then, in the next step (Step 12) the treatment solution is drained from the central tank 7 and the central tank 7 is filled with fresh water (Step 13). Finally, the central pump 21 is operated in the supply mode so that each consumer assembly 5, 5' is supplied fresh water which is then used to flush the buffer tanks 37, lines and supply devices of the consumer assemblies (Step 14).

The system 1 with the control unit 19 being configured so as to control it in the above-described manner provides for the following advantages. The flush with treatment solution can be carried out without the need for additional equipment and no ground service involving service personnel is required.

The treatment process can be carried out with minimal manual intervention required, and may require only of the user the low-skilled task of providing a treatment medium into the system, so that it may be performed so as to minimise any operational interruptions, i.e., it can be performed on ground and in principle also during flight. Additionally, when the method is carried out there is a reduced need to heat water and/or the treatment solution as compared to thermal treatment methods.

### Reference numerals:

- 1: water supply and distribution system
- 3: aircraft
- 5: first consumer assembly
- 5': second consumer assembly
- 7: central water tank
- 9: connector
- 9': valve
- 11: supply line
- 13: horizontal
- 15: fill/drain coupling
- 17: valve
- 19: control unit
- 20: communication device
- 21: central pump
- 23: upstream side - pump
- 25: downstream side - pump
- 27: high pressure conduit system
- 29: conduit
- 31: sink
- 33: faucet
- 35: toilet
- 37: buffer tank
- 39: inlet
- 41: outlet
- 42: micro pump
- 43: toilet valve
- 44: overflow line
- 45: inlet valve
- 46: level sensor
- 46': temperature sensor
- 47: heater
- 48: treatment inlet
- 49: galley device
- 51: fluid disperser
- 51': valve

## Claims

1. A method for operating an on-board water supply and distribution system (1) of an aircraft (3) for supplying water, the system comprising
a central water tank (7),
at least one first consumer assembly (5), each of the at least one first consumer assemblies (5) comprising a supply device and a buffer tank (37) and each of the at least one first consumer assemblies (5) being configured to supply fluid from the buffer tank (37) via the supply device,
wherein each of the at least one first consumer assemblies (5) comprises a treatment inlet (48) configured for receiving a treatment medium and for transferring the treatment medium into the respective buffer tank (37),
a pump (21) having an upstream side (23) and a downstream side (25) and being configured such that it may operate in a supply mode and a reverse mode, and
a high pressure conduit system (27),
wherein the central water tank (7) is connected to the upstream side (23) of the pump (21),
wherein the high pressure conduit system (27) connects the downstream side (25) with the at least one first consumer assembly (5), with the conduit system (27) being configured such that the pump (21), when operating in the supply mode, is capable of supplying fluid from the downstream side (25) to the buffer tank (37) of each of the at least one first consumer assemblies (5),
wherein, when the pump (21) is operating in the reverse mode, it is capable of drawing fluid from the high pressure conduit system (27) towards the upstream side (23),
the method comprising the following steps:
operating the pump (21) in the supply mode so that water from the central tank (7) is supplied to at least one of the at least one first consumer assemblies (5) wherein the buffer tank (37) of the at least one of the first consumer assemblies (5) is filled to a predetermined fill level,
providing a treatment medium to the buffer tank (37) via the treatment inlet (48) so as to mix with water in the buffer tank (37) to form a treatment solution,
and
operating the pump (21) in the reverse mode so that the treatment solution in the buffer tank (37) of the at least one of the first consumer (5) assemblies passes through a part of the high pressure conduit system (27) and the upstream side (23) of the pump (21).

2. The method according to claim 1, wherein each of the buffer tanks (37) of the at least one first consumer assemblies (5) comprises the treatment inlet (48) and/or
wherein the treatment inlet (48') is provided in a conduit section connecting the respective buffer tank (37) and the high pressure conduit system (27).

3. The method according to any of claims 1 to 2, wherein the supply device of each of the at least one first consumer assemblies (5) is operated such that the treatment solution is supplied via the respective supply device.

4. The method according to any of claims 1 to 3, wherein the system comprises a plurality of first consumer assemblies (5), and each of the plurality of first consumer assemblies (5) is provided with an inlet valve (45), which is arranged between the buffer tank (37) and the high pressure conduit system (27), each of the inlet valves (45) having a closed position and an open position,
wherein in the step of operating the pump (21) in the supply mode, water from the central tank (7) is supplied to the plurality of the first consumer assemblies (5) wherein the buffer tanks (37) of the plurality of the first consumer assemblies (5) are filled to a predetermined fill level,
wherein the treatment medium is provided to the buffer tank (37) of the plurality of first consumer assemblies (5) via the respective treatment inlet (48) to mix with water in the buffer tanks (37) to form a treatment solution,
and
wherein in the step of operating the pump (21) in the reverse mode, the inlet valves (45) of the plurality of first consumer assemblies (5) are controlled to be in the open position so that the treatment solution in the buffer tanks (37) of the plurality of first consumer assemblies passes through a part of the high pressure conduit system and the upstream side of the pump (21).

5. The method according to any of claims 1 to **4,** wherein the connection between the central water tank (7) and the upstream side of the pump (21) comprises a fill/drain coupling (15) and
wherein when operating the pump (21) in the reverse mode so that the treatment solution in the buffer tank (37) of the at least one of the first consumer assemblies (5) passes through a part of the high pressure conduit system (27) and the upstream side of the pump (21), the treatment solution is passed through the fill/drain coupling (15) and/or to the central water tank (7).

6. The method according to any of claims 1 to 5, wherein the system further comprises second consumer assemblies, each second consumer assembly (5') comprising a supply device and a buffer tank (37) and each second consumer assembly (5') being configured to supply fluid from the buffer tank (37) via the supply device,
wherein the high pressure conduit system (27) connects the downstream side of the pump (21) with the plurality of second consumer assemblies (5'), with the conduit system (27) being configured such that the pump (21), when operating in the supply mode, is capable of supplying fluid from the downstream (25) side to the second consumer assemblies (5'), so that the fluid is received in the buffer tank (37) of at least one of the second consumer assemblies (5'),
wherein when the pump (21) is operated in the reverse mode so that the treatment solution in the buffer tank (37) of the at least one of the first consumer assemblies (5) passes through a part of the high pressure conduit system (27) and the upstream side (23) of the pump (21), the treatment solution is passed to the central water tank (7), and
wherein the method comprises the following further step of operating the pump (21) in the supply mode so that treatment solution from the central tank (7) is supplied to at least one of the second consumer assemblies (5'), the treatment solution being received in the buffer tank (37) of at least one of the second consumer assemblies (5').

7. The method according to any preceding claim, comprising filling the buffer tank (37) to a maximum fill level once the treatment medium has been provided to the buffer tank (37).

8. The method according to any preceding claim, comprising operating the pump (21) in
the reverse mode such that the treatment solution in the buffer tank (37) of the at least one of the first consumer assemblies (5) is passed to the central water tank (7), and
subsequently draining the central tank (7), and
flushing the central water tank (7), each buffer tank (37) and the high pressure conduit system (27) with water.

9. An on-board water supply and distribution system (1) of an aircraft (3) for supplying water, the system comprising
a central water tank (7),
at least one first consumer assembly (5), each of the at least one first consumer assemblies (5) comprising a supply device and a buffer tank (37) and each of the at least one first consumer assemblies (5) being configured to supply fluid from the buffer tank (37) via the supply device,
wherein each of the at least one first consumer assemblies (5) comprises a treatment inlet (48) for receiving a treatment medium and for transferring the treatment medium into the respective buffer tank (37),
a pump (21) having an upstream side (23) and a downstream side (25) and being configured such that it may operate in a supply mode and a reverse mode,
a high pressure conduit system (27), and
a control unit (19),
wherein the central water tank (7) is connected to the upstream side (23) of the pump (21),
wherein the high pressure conduit system (27) connects the downstream side (25) with the at least one first consumer assembly (5), with the conduit system (27) being configured such that the pump (21), when operating in the supply mode, is capable of supplying fluid from the downstream side (25) to the buffer tank (37) of each of the first consumer assemblies (5),
wherein, when the pump (21) is operating in the reverse mode, it is capable of drawing fluid from the high pressure conduit system (27) towards the upstream side (23),
the control unit (19) being connected to the pump (21) and each of the at least one first consumer assemblies (5) and being configured such that
in a first step the control unit (19) operates the pump (21) in the supply mode so that water from the central tank (7) is supplied to at least one of the at least one first consumer assemblies (5) wherein the buffer tank (37) of the at least one of the at least one first consumer assemblies (5) is filled to a predetermined fill level,
in a second step a treatment medium is provided to the buffer tank (37) of the at least one of the first consumer assemblies (5) to mix with water in the buffer tank (37) to form a treatment solution, and
in a third step the control unit (19) operates the pump (21) in the reverse mode so the treatment solution in the buffer tank (37) of the at least one of the first consumer assemblies (5) passes through a part of the high pressure conduit system (27) and the upstream side of the pump.

10. The system according to claim 9, wherein each of the buffer tanks (37) of the at least one first consumer assemblies (5) comprises the treatment inlet (48) and/or
wherein the treatment inlet (48') is provided in a conduit section connecting the respective buffer tank (37) and the high pressure conduit system (27).

11. The system according to claim 9 or 10, wherein the control unit (19) is configured such that the supply device of each of the at least one first consumer assemblies (5) is operated such that the treatment solution is supplied via the respective supply device.

12. The system according to any of claims 9 to 11, comprising a plurality of first consumer assemblies, and wherein each of the plurality of first consumer assemblies (5) is provided with an inlet valve (45), which is arranged between the buffer tank (37) and the high pressure conduit system (27), each of the inlet valves (45) having a closed position and an open position,
wherein the control unit (19) is connected to each of the inlet valves (45) of the plurality of first consumer assemblies (5), so as to control the position of the inlet valves (45),
wherein the control unit (19) is further configured such that
in the first step the control unit (19) operates the pump (21) in the supply mode, so that water from the central tank (7) is supplied to the plurality of first consumer assemblies (5) wherein the buffer tanks (37) of the plurality of first consumer assemblies (5) are filled to a predetermined fill level,
in the second step the treatment medium is provided into the buffer tanks (37) of the plurality of first consumer assemblies (5) via the respective treatment inlet (48) to mix with water in the buffer tank (37) to form a treatment solution, and
in the third step the control unit (19) operates the pump (21) in the reverse mode,
wherein the control unit controls the inlet valves (45) of the plurality of first consumer assemblies such that the inlet valves (45) thereof are in the open position so that the treatment solution in the buffer tanks (37) of the plurality of first consumer assemblies passes through a part of the high pressure conduit system and the upstream side of the pump (21).

13. The system according to any of claims 9 to 12, wherein the connection between the central water tank (7) and the upstream side (23) of the pump (21) comprises a fill/drain coupling (15) having a fill/drain valve (17) which has an open position and a closed position and is operatively connected to the control unit (19),
wherein the control unit (19) is configured such that when it operates the pump (21) in the reverse mode so that the treatment solution in the buffer tank (37) of the at least one of the first consumer assemblies (5) passes through a part of the high pressure conduit system (27) and the upstream side (23) of the pump (21), the control unit (19) controls the fill/drain valve (17) such that
it is in the open position and the treatment solution is passed through the fill/drain coupling (15) or
it is in the closed position and the treatment solution is passed to the central water tank (7).

14. The system according to any of claims 9 to 13, further comprising second consumer assemblies (5'), each second consumer assembly (5') comprising a supply device and a buffer tank (37) and each second consumer assembly (5') being configured to supply fluid from the buffer tank (37) via the supply device,
wherein the high pressure conduit system (27) connects the downstream side (25) of the pump with the plurality of second consumer assemblies (5'), with the conduit system (27) being configured such that the pump (21), when operating in the supply mode, is capable of supplying fluid from the downstream side (25) to the second consumer assemblies (5'), so that the fluid is received in the buffer tank (37) of at least one of the second consumer assemblies (5'),
wherein the control unit (19) is configured such that
when in the third step the control unit (19) operates the pump (21) in the reverse mode so that the treatment solution in the buffer tank (37) of the at least one of the first consumer assemblies (5) passes through a part of the high pressure conduit system (27) and the upstream side of the pump (21), the treatment solution is passed to the central water tank (7),
in a fourth step the control unit (19) operates the pump (21) in the supply mode so that treatment solution from the central tank is supplied to at least one of the second consumer assemblies (5'), the treatment solution being received in the buffer tank (37) of at least one of the second consumer assemblies (5').

15. Aircraft (3) comprising a system according to any of claims 9 to 14

## Patentansprüche

1. Verfahren zum Betreiben eines bordeigenen Wasserversorgungs- und Verteilungssystems (1) eines Flugzeugs (3) zum Zuführen von Wasser, wobei das System Folgendes umfasst:
einen zentralen Wassertank (7),
mindestens eine erste Verbraucherbaugruppe (5), wobei jede der mindestens einen ersten Verbraucherbaugruppe (5) eine Versorgungsvorrichtung und einen Puffertank (37) umfasst und jede der mindestens einen ersten Verbraucherbaugruppe (5) dazu ausgelegt ist, Fluid aus dem Puffertank (37) über die Versorgungsvorrichtung zuzuführen,
wobei jede der mindestens einen ersten Verbraucherbaugruppe (5) einen Behandlungseinlass (48) umfasst, der zum Aufnehmen eines Behandlungsmediums und zum Überführen des Behandlungsmediums in den jeweiligen Puffertank (37) ausgelegt ist,
eine Pumpe (21), die eine stromaufwärtige Seite (23) und eine stromabwärtige Seite (25) aufweist und so ausgelegt ist, dass sie in einem Versorgungsmodus und einem Rückwärtsmodus arbeiten kann, und
ein Hochdruckleitungssystem (27),
wobei der zentrale Wassertank (7) mit der stromaufwärtigen Seite (23) der Pumpe (21) verbunden ist,
wobei das Hochdruckleitungssystem (27) die stromabwärtige Seite (25) mit der mindestens einen ersten Verbraucherbaugruppe (5) verbindet, wobei das Leitungssystem (27) so ausgelegt ist, dass die Pumpe (21), wenn sie im Versorgungsmodus arbeitet, in der Lage ist, dem Puffertank (37) jeder der mindestens einen ersten Verbraucherbaugruppe (5) Fluid von der stromabwärtigen Seite (25) zuzuführen,
wobei, wenn die Pumpe (21) im Rückwärtsmodus arbeitet, sie in der Lage ist, Fluid aus dem Hochdruckleitungssystem (27) zu der stromaufwärtigen Seite (23) zu saugen,
wobei das Verfahren die folgenden Schritte umfasst:
Betreiben der Pumpe (21) im Versorgungsmodus, so dass Wasser aus dem zentralen Tank (7) mindestens einer der mindestens einen ersten Verbraucherbaugruppe (5) zugeführt wird, wobei der Puffertank (37) der mindestens einen der ersten Verbraucherbaugruppen (5) auf einen vorbestimmten Füllstand gefüllt wird,
Versehen des Puffertanks (37) mit einem Behandlungsmedium über den Behandlungseinlass (48), um es in dem Puffertank (37) mit Wasser zu mischen, um eine Behandlungslösung zu bilden,
und
Betreiben der Pumpe (21) im Rückwärtsmodus, so dass die Behandlungslösung im Puffertank (37) der mindestens einen der ersten Verbraucherbaugruppe (5) durch einen Teil des Hochdruckleitungssystems (27) und die stromaufwärtige Seite (23) der Pumpe (21) hindurchgeht.

2. Verfahren nach Anspruch 1, wobei jeder der Puffertanks (37) der mindestens einen ersten Verbraucherbaugruppe (5) den Behandlungseinlass (48) umfasst und/oder
wobei der Behandlungseinlass (48') in einem Leitungsabschnitt vorgesehen ist, der den jeweiligen Puffertank (37) und das Hochdruckleitungssystem (27) verbindet.

3. Verfahren nach einem der Ansprüche 1 bis 2, wobei die Versorgungsvorrichtung jeder der mindestens einen ersten Verbraucherbaugruppe (5) so betrieben wird, dass die Behandlungslösung über die jeweilige Versorgungsvorrichtung zugeführt wird.

4. Verfahren nach einem der Ansprüche 1 bis 3, wobei das System mehrere erste Verbraucherbaugruppen (5) umfasst und jede der mehreren ersten Verbraucherbaugruppen (5) mit einem Einlassventil (45) versehen ist, das zwischen dem Puffertank (37) und dem Hochdruckleitungssystem (27) angeordnet ist, wobei jedes der Einlassventile (45) eine geschlossene Position und eine offene Position aufweist,
wobei in dem Schritt des Betreibens der Pumpe (21) im Versorgungsmodus Wasser aus dem zentralen Tank (7) den mehreren der ersten Verbraucherbaugruppen (5) zugeführt wird, wobei die Puffertanks (37) der mehreren der ersten Verbraucherbaugruppen (5) auf einen vorbestimmten Füllstand gefüllt werden,
wobei der Puffertank (37) der mehreren ersten Verbraucherbaugruppen (5) über den jeweiligen Behandlungseinlass (48) mit dem Behandlungsmedium versehen wird, um es in den Puffertanks (37) mit Wasser zu mischen, um eine Behandlungslösung zu bilden,
und
wobei in dem Schritt des Betreibens der Pumpe (21) im Rückwärtsmodus die Einlassventile (45) der mehreren ersten Verbraucherbaugruppen (5) so gesteuert werden, dass sie sich in der offenen Position befinden, so dass die Behandlungslösung in den Puffertanks (37) der mehreren ersten Verbraucherbaugruppen durch einen Teil des Hochdruckleitungssystems und die stromaufwärtige Seite der Pumpe (21) hindurchgeht.

5. Verfahren nach einem der Ansprüche 1 bis 4, wobei die Verbindung zwischen dem zentralen Wassertank (7) und der stromaufwärtigen Seite der Pumpe (21) eine Füll-/Abflusskupplung (15) umfasst und
wobei beim Betreiben der Pumpe (21) im Rückwärtsmodus, so dass die Behandlungslösung in dem Puffertank (37) der mindestens einen der ersten Verbraucherbaugruppen (5) durch einen Teil des Hochdruckleitungssystems (27) und die stromaufwärtige Seite der Pumpe (21) hindurchgeht, die Behandlungslösung durch die Füll-/Abflusskupplung (15) und/oder zu dem zentralen Wassertank (7) geleitet wird.

6. Verfahren nach einem der Ansprüche 1 bis 5, wobei das System ferner zweite Verbraucherbaugruppen umfasst, wobei jede zweite Verbraucherbaugruppe (5') eine Versorgungsvorrichtung und einen Puffertank (37) umfasst und jede zweite Verbraucherbaugruppe (5') dazu ausgelegt ist, Fluid aus dem Puffertank (37) über die Versorgungsvorrichtung zuzuführen,
wobei das Hochdruckleitungssystem (27) die stromabwärtige Seite der Pumpe (21) mit den mehreren zweiten Verbraucherbaugruppen (5') verbindet, wobei das Leitungssystem (27) so ausgelegt ist, dass die Pumpe (21), wenn sie im Versorgungsmodus arbeitet, in der Lage ist, Fluid von der stromabwärtigen (25) Seite den zweiten Verbraucherbaugruppen (5') zuzuführen, so dass das Fluid in dem Puffertank (37) mindestens einer der zweiten Verbraucherbaugruppen (5') aufgenommen wird,
wobei, wenn die Pumpe (21) im Rückwärtsmodus betrieben wird, so dass die Behandlungslösung in dem Puffertank (37) der mindestens einen der ersten Verbraucherbaugruppen (5) durch einen Teil des Hochdruckleitungssystems (27) und die stromaufwärtige Seite (23) der Pumpe (21) hindurchgeht, die Behandlungslösung zu dem zentralen Wassertank (7) geleitet wird, und
wobei das Verfahren den folgenden weiteren Schritt des Betreibens der Pumpe (21) im Versorgungsmodus umfasst, so dass die Behandlungslösung aus dem zentralen Tank (7) mindestens einer der zweiten Verbraucherbaugruppen (5') zugeführt wird, wobei die Behandlungslösung in dem Puffertank (37) mindestens einer der zweiten Verbraucherbaugruppen (5') aufgenommen wird.

7. Verfahren nach einem der vorhergehenden Ansprüche, umfassend das Füllen des Puffertanks (37) bis zu einem maximalen Füllstand, sobald der Puffertank (37) mit dem Behandlungsmedium versehen wurde.

8. Verfahren nach einem der vorhergehenden Ansprüche, umfassend das Betreiben der Pumpe (21) im Rückwärtsmodus, so dass die Behandlungslösung in dem Puffertank (37) der mindestens einen der ersten Verbraucherbaugruppen (5) zu dem zentralen Wassertank (7) geleitet wird, und
anschließend das Entleeren des zentralen Tanks (7) und
das Spülen des zentralen Wassertanks (7), jedes Puffertanks (37) und des Hochdruckleitungssystems (27) mit Wasser.

9. Bordeigenes Wasserversorgungs- und Verteilungssystem (1) eines Flugzeugs (3) zum Zuführen von Wasser, wobei das System Folgendes umfasst:
einen zentralen Wassertank (7),
mindestens eine erste Verbraucherbaugruppe (5), wobei jede der mindestens einen ersten Verbraucherbaugruppe (5) eine Versorgungsvorrichtung und einen Puffertank (37) umfasst und jede der mindestens einen ersten Verbraucherbaugruppe (5) dazu ausgelegt ist, Fluid aus dem Puffertank (37) über die Versorgungsvorrichtung zuzuführen,
wobei jede der mindestens einen ersten Verbraucherbaugruppe (5) einen Behandlungseinlass (48) zum Aufnehmen eines Behandlungsmediums und zum Überführen des Behandlungsmediums in den jeweiligen Puffertank (37) umfasst,
eine Pumpe (21), die eine stromaufwärtige Seite (23) und eine stromabwärtige Seite (25) aufweist und so ausgelegt ist, dass sie in einem Versorgungsmodus und einem Rückwärtsmodus arbeiten kann,
ein Hochdruckleitungssystem (27) und
eine Steuereinheit (19),
wobei der zentrale Wassertank (7) mit der stromaufwärtigen Seite (23) der Pumpe (21) verbunden ist,
wobei das Hochdruckleitungssystem (27) die stromabwärtige Seite (25) mit der mindestens einen ersten Verbraucherbaugruppe (5) verbindet, wobei das Leitungssystem (27) so ausgelegt ist, dass die Pumpe (21), wenn sie im Versorgungsmodus arbeitet, in der Lage ist, Fluid von der stromabwärtigen Seite (25) dem Puffertank (37) jeder der ersten Verbraucherbaugruppen (5) zuzuführen,
wobei, wenn die Pumpe (21) im Rückwärtsmodus arbeitet, sie in der Lage ist, Fluid aus dem Hochdruckleitungssystem (27) zu der stromaufwärtigen Seite (23) zu saugen,
wobei die Steuereinheit (19) mit der Pumpe (21) und jeder der mindestens einen ersten Verbraucherbaugruppen (5) verbunden ist und so ausgelegt ist, dass
in einem ersten Schritt die Steuereinheit (19) die Pumpe (21) im Versorgungsmodus betreibt, sodass Wasser aus dem zentralen Tank (7) mindestens einer der mindestens einen ersten Verbraucherbaugruppen (5) zugeführt wird, wobei der Puffertank (37) der mindestens einen der mindestens einen ersten Verbraucherbaugruppen (5) auf einen vorbestimmten Füllstand gefüllt wird,
in einem zweiten Schritt der Puffertank (37) der mindestens einen der ersten Verbraucherbaugruppen (5) mit einem Behandlungsmedium versehen wird, um es in dem Puffertank (37) mit Wasser zu mischen, um eine Behandlungslösung zu bilden, und
in einem dritten Schritt die Steuereinheit (19) die Pumpe (21) im Rückwärtsmodus betreibt, so dass die Behandlungslösung im Puffertank (37) der mindestens einen der ersten Verbraucherbaugruppen (5) einen Teil des Hochdruckleitungssystems (27) und die stromaufwärtige Seite der Pumpe durchläuft.

10. System nach Anspruch 9, wobei jeder der Puffertanks (37) der mindestens einen ersten Verbraucherbaugruppe (5) den Behandlungseinlass (48) umfasst und/oder
wobei der Behandlungseinlass (48') in einem Leitungsabschnitt vorgesehen ist, der den jeweiligen Puffertank (37) und das Hochdruckleitungssystem (27) verbindet.

11. System nach Anspruch 9 oder 10, wobei die Steuereinheit (19) so ausgelegt ist, dass die Versorgungsvorrichtung jeder der mindestens einen ersten Verbraucherbaugruppe (5) so betrieben wird, dass die Behandlungslösung über die jeweilige Versorgungsvorrichtung zugeführt wird.

12. System nach einem der Ansprüche 9 bis 11, umfassend mehrere erste Verbraucherbaugruppen und wobei jede der mehreren ersten Verbraucherbaugruppen (5) mit einem Einlassventil (45) versehen ist, das zwischen dem Puffertank (37) und dem Hochdruckleitungssystem (27) angeordnet ist, wobei jedes der Einlassventile (45) eine geschlossene Position und eine offene Position aufweist, wobei die Steuereinheit (19) mit jedem der Einlassventile (45) der mehreren ersten Verbraucherbaugruppen (5) verbunden ist, um die Position der Einlassventile (45) zu steuern,
wobei die Steuereinheit (19) ferner so ausgelegt ist, dass
im ersten Schritt die Steuereinheit (19) die Pumpe (21) im Versorgungsmodus betreibt, so dass Wasser aus dem zentralen Tank (7) den mehreren ersten Verbraucherbaugruppen (5) zugeführt wird, wobei die Puffertanks (37) der mehreren ersten Verbraucherbaugruppen (5) auf einen vorbestimmten Füllstand gefüllt werden,
im zweiten Schritt das Behandlungsmedium über den jeweiligen Behandlungseinlass (48) in die Pufferbehälter (37) der mehreren ersten Verbraucherbaugruppen (5) eingebracht wird, um sich mit Wasser im Pufferbehälter (37) zu einer Behandlungslösung zu vermischen, und
im dritten Schritt die Steuereinheit (19) die Pumpe (21) im Rückwärtsmodus betreibt,
wobei die Steuereinheit die Einlassventile (45) der mehreren ersten Verbraucherbaugruppen so steuert, dass sich die Einlassventile (45) davon in der offenen Position befinden, sodass die Behandlungslösung in den Puffertanks (37) der mehreren ersten Verbraucherbaugruppen durch einen Teil des Hochdruckleitungssystems und die stromaufwärtige Seite der Pumpe (21) hindurchgeht.

13. System nach einem der Ansprüche 9 bis 12, wobei die Verbindung zwischen dem zentralen Wassertank (7) und der stromaufwärtigen Seite (23) der Pumpe (21) eine Füll-/Ablasskupplung (15) mit einem Füll-/Ablassventil (17) umfasst, das eine offene Position und eine geschlossene Position aufweist und mit der Steuereinheit (19) wirkverbunden ist,
wobei die Steuereinheit (19) so ausgelegt ist, dass, wenn sie die Pumpe (21) im Rückwärtsmodus betreibt, so dass die Behandlungslösung in dem Puffertank (37) der mindestens einen der ersten Verbraucherbaugruppen (5) durch einen Teil des Hochdruckleitungssystems (27) und die stromaufwärtige Seite (23) der Pumpe (21) verläuft, die Steuereinheit (19) das Füll-/Ablassventil (17) so steuert, dass
es sich in der offenen Position befindet und die Behandlungslösung durch die Füll-/Ablaufkupplung (15) geleitet wird oder
es sich in der geschlossenen Position befindet und die Behandlungslösung zu dem zentralen Wassertank (7) geleitet wird.

14. System nach einem der Ansprüche 9 bis 13, ferner umfassend zweite Verbraucherbaugruppen (5'), wobei jede zweite Verbraucherbaugruppe (5') eine Versorgungsvorrichtung und einen Puffertank (37) umfasst und jede zweite Verbraucherbaugruppe (5') dazu ausgelegt ist, Fluid aus dem Puffertank (37) über die Versorgungsvorrichtung zuzuführen,
wobei das Hochdruckleitungssystem (27) die stromabwärtige Seite (25) der Pumpe mit den mehreren zweiten Verbraucherbaugruppen (5') verbindet, wobei das Leitungssystem (27) so ausgelegt ist, dass die Pumpe (21), wenn sie im Versorgungsmodus arbeitet, in der Lage ist, Fluid von der stromabwärtigen Seite (25) den zweiten Verbraucherbaugruppen (5') zuzuführen, so dass das Fluid in dem Puffertank (37) mindestens einer der zweiten Verbraucherbaugruppen (5') aufgenommen wird,
wobei die Steuereinheit (19) so ausgelegt ist, dass
wenn im dritten Schritt die Steuereinheit (19) die Pumpe (21) im Rückwärtsmodus betreibt, so dass die Behandlungslösung im Puffertank (37) der mindestens einen der ersten Verbraucherbaugruppen (5) einen Teil des Hochdruckleitungssystems (27) und die stromaufwärtige Seite der Pumpe (21) durchläuft, die Behandlungslösung zu dem zentralen Wassertank (7) geleitet wird,
in einem vierten Schritt die Steuereinheit (19) die Pumpe (21) im Versorgungsmodus betreibt, so dass die Behandlungslösung aus dem zentralen Tank mindestens einer der zweiten Verbraucherbaugruppen (5') zugeführt wird, wobei die Behandlungslösung in dem Puffertank (37) mindestens einer der zweiten Verbraucherbaugruppen (5') aufgenommen wird.

15. Flugzeug (3), das ein System nach einem der Ansprüche 9 bis 14 umfasst.

## Revendications

1. Procédé de fonctionnement d'un système d'alimentation et de distribution d'eau (1) à bord d'un aéronef (3) destiné à fournir de l'eau, le système comprenant
un réservoir d'eau central (7),
au moins un premier ensemble consommateur (5), chacun du ou des premiers ensembles consommateurs (5) comprenant un dispositif d'alimentation et un réservoir tampon (37) et chacun du ou des premiers ensembles consommateurs (5) étant conçu pour fournir du fluide depuis le réservoir tampon (37) par l'intermédiaire du dispositif d'alimentation,
chacun du ou des premiers ensembles consommateurs (5) comprenant une entrée de traitement (48) conçue pour recevoir un milieu de traitement et pour transférer le milieu de traitement dans le réservoir tampon (37) respectif,
une pompe (21) présentant un côté amont (23) et un côté aval (25) et étant conçue de sorte qu'elle puisse fonctionner en mode alimentation et en mode inversé, et un système de conduite haute pression (27),
le réservoir d'eau central (7) étant raccordé au côté amont (23) de la pompe (21),
le système de conduite haute pression (27) raccordant le côté aval (25) à l'au moins un premier ensemble consommateur (5), le système de conduite (27) étant conçu de sorte que la pompe (21), lorsqu'elle fonctionne en mode alimentation, soit apte à fournir du fluide provenant du côté aval (25) au réservoir tampon (37) de chacun du ou des premiers ensembles consommateurs (5),
lorsqu'elle fonctionne en mode inversé, la pompe (21) étant apte à aspirer du fluide depuis le système de conduite haute pression (27) vers le côté amont (23),
le procédé comprenant les étapes consistant à :
faire fonctionner la pompe (21) en mode alimentation de sorte que l'eau provenant du réservoir central (7) soit fournie à au moins un parmi le ou les premiers ensembles consommateurs (5), le réservoir tampon (37) du ou des premiers ensembles consommateurs (5) étant rempli jusqu'à un niveau de remplissage prédéfini,
fournir un milieu de traitement au réservoir tampon (37) par l'intermédiaire de l'entrée de traitement (48) de sorte à le mélanger avec de l'eau dans le réservoir tampon (37) pour former une solution de traitement,
et
faire fonctionner la pompe (21) en mode inversé de sorte que la solution de traitement dans le réservoir tampon (37) du ou des premiers ensembles consommateurs (5) passe à travers une partie du système de conduite haute pression (27) et le côté amont (23) de la pompe (21).

2. Procédé selon la revendication 1, chacun des réservoirs tampons (37) de l'au moins un parmi les premiers ensembles consommateurs (5) comprenant l'entrée de traitement (48) et/ou
l'entrée de traitement (48') étant située dans une section de conduit raccordant le réservoir tampon (37) respectif et le système de conduit haute pression (27).

3. Procédé selon l'une quelconque des revendications 1 et 2, le dispositif d'alimentation de chacun du ou des premiers ensembles consommateurs (5) fonctionnant de sorte que la solution de traitement soit fournie par l'intermédiaire du dispositif d'alimentation respectif.

4. Procédé selon l'une quelconque des revendications 1 à 3, le système comprenant une pluralité de premiers ensembles consommateurs (5), et chacun de la pluralité de premiers ensembles consommateurs (5) étant pourvu d'une vanne d'entrée (45) qui est disposée entre le réservoir tampon (37) et le système de conduite haute pression (27), chacune des vannes d'entrée (45) ayant une position fermée et une position ouverte,
à l'étape de fonctionnement de la pompe (21) en mode alimentation, de l'eau provenant du réservoir central (7) étant fournie à la pluralité des premiers ensembles consommateurs (5), les réservoirs tampons (37) de la pluralité des premiers ensembles consommateurs (5) étant remplis jusqu'à un niveau de remplissage prédéfini,
le milieu de traitement étant fourni au réservoir tampon (37) de la pluralité de premiers ensembles consommateurs (5) par l'intermédiaire de l'entrée de traitement (48) respective pour le mélanger à l'eau dans les réservoirs tampons (37) pour former une solution de traitement,
et
à l'étape de fonctionnement de la pompe (21) en mode inversé, les vannes d'entrée (45) de la pluralité de premiers ensembles consommateurs (5) étant commandées pour être en position ouverte de sorte que la solution de traitement dans les réservoirs tampons (37) de la pluralité de premiers ensembles consommateurs passe à travers une partie du système de conduite haute pression et le côté amont de la pompe (21).

5. Procédé selon l'une quelconque des revendications 1 à 4, le raccordement entre le réservoir d'eau central (7) et le côté amont de la pompe (21) comprenant un raccord de remplissage/vidange (15) et
lors du fonctionnement de la pompe (21) en mode inversé de sorte que la solution de traitement dans le réservoir tampon (37) du ou des premiers ensembles consommateurs (5) passe à travers une partie du système de conduite haute pression (27) et le côté amont de la pompe (21), la solution de traitement passant à travers le raccord de remplissage/vidange (15) et/ou vers le réservoir d'eau central (7).

6. Procédé selon l'une quelconque des revendications 1 à 5, le système comprenant en outre des seconds ensembles consommateurs, chaque second ensemble consommateur (5') comprenant un dispositif d'alimentation et un réservoir tampon (37), et chaque second ensemble consommateur (5') étant conçu pour fournir du fluide depuis le réservoir tampon (37) par l'intermédiaire du dispositif d'alimentation,
le système de conduite haute pression (27) raccordant le côté aval de la pompe (21) à la pluralité de seconds ensembles consommateurs (5'), le système de conduite (27) étant conçu de sorte que la pompe (21), lorsqu'elle fonctionne en mode alimentation, soit apte à fournir du fluide provenant du côté aval (25) aux seconds ensembles consommateurs (5'), de sorte que le fluide soit reçu dans le réservoir tampon (37) du ou des seconds ensembles consommateurs (5'),
lorsque la pompe (21) fonctionne en mode inversé de sorte que la solution de traitement dans le réservoir tampon (37) du ou des premiers ensembles consommateurs (5) passe à travers une partie du système de conduite haute pression (27) et le côté amont (23) de la pompe (21), la solution de traitement passant dans le réservoir d'eau central (7), et
le procédé comprenant l'étape supplémentaire suivante consistant à faire fonctionner la pompe (21) en mode alimentation de sorte que la solution de traitement provenant du réservoir central (7) soit fournie au ou aux seconds ensembles consommateurs (5'), la solution de traitement étant reçue dans le réservoir tampon (37) du ou des seconds ensembles consommateurs (5').

7. Procédé selon l'une quelconque des revendications précédentes, comprenant l'étape consistant à remplir le réservoir tampon (37) jusqu'à un niveau de remplissage maximum une fois que le milieu de traitement a été fourni au réservoir tampon (37).

8. Procédé selon l'une quelconque des revendications précédentes, comprenant les étapes consistant à faire fonctionner la pompe (21) en mode inversé de sorte que la solution de traitement dans le réservoir tampon (37) du ou des premiers ensembles consommateurs (5) soit amenée au réservoir d'eau central (7), et
vidanger ensuite le réservoir central (7), et
rincer à l'eau le réservoir d'eau central (7), chaque réservoir tampon (37) et le système de conduite haute pression (27).

9. Système d'alimentation et de distribution d'eau (1) à bord d'un aéronef (3) destiné à fournir de l'eau, le système comprenant
un réservoir d'eau central (7),
au moins un premier ensemble consommateur (5), chacun du ou des premiers ensembles consommateurs (5) comprenant un dispositif d'alimentation et un réservoir tampon (37) et chacun du ou des premiers ensembles consommateurs (5) étant conçu pour fournir du fluide depuis le réservoir tampon (37) par l'intermédiaire du dispositif d'alimentation,
chacun du ou des premiers ensembles consommateurs (5) comprenant une entrée de traitement (48) pour recevoir un milieu de traitement et pour transférer le milieu de traitement dans le réservoir tampon (37) respectif,
une pompe (21) présentant un côté amont (23) et un côté aval (25) et étant conçue de sorte qu'elle puisse fonctionner en mode alimentation et en mode inversé,
un système de conduite haute pression (27), et
une unité de commande (19),
le réservoir d'eau central (7) étant raccordé au côté amont (23) de la pompe (21),
le système de conduite haute pression (27) raccordant le côté aval (25) à l'au moins un premier ensemble consommateur (5), le système de conduite (27) étant conçu de sorte que la pompe (21), lorsqu'elle fonctionne en mode alimentation, soit apte à fournir du fluide provenant du côté aval (25) au réservoir tampon (37) de chacun des premiers ensembles consommateurs (5),
lorsqu'elle fonctionne en mode inversé, la pompe (21) étant apte à aspirer du fluide depuis le système de conduite haute pression (27) vers le côté amont (23),
l'unité de commande (19) étant reliée à la pompe (21) et à chacun du ou des premiers ensembles consommateurs (5) et étant conçue de sorte que
dans une première étape, l'unité de commande (19) fait fonctionner la pompe (21) en mode alimentation de sorte que l'eau provenant du réservoir central (7) soit fournie à au moins un parmi le ou les premiers ensembles consommateurs (5), le réservoir tampon (37) de l'au moins un parmi le ou les premiers ensembles consommateurs (5) étant rempli jusqu'à un niveau de remplissage prédéfini, dans une deuxième étape, un milieu de traitement étant fourni au réservoir tampon (37) du premier ensemble consommateur (5) pour le mélanger à l'eau dans le réservoir tampon (37) afin de former une solution de traitement
dans une troisième étape, l'unité de commande (19) faisant fonctionner la pompe (21) en mode inversé, de sorte que la solution de traitement dans le réservoir tampon (37) du ou des premiers ensembles consommateurs (5) passe à travers une partie du système de conduite haute pression (27) et le côté amont de la pompe.

10. Système selon la revendication 9, chacun des réservoirs tampons (37) de l'au moins un parmi les premiers ensembles consommateurs (5) comprenant l'entrée de traitement (48) et/ou
l'entrée de traitement (48') étant située dans une section de conduit raccordant le réservoir tampon (37) respectif et le système de conduit haute pression (27).

11. Système selon la revendication 9 ou 10, l'unité de commande (19) étant conçue de sorte que le dispositif d'alimentation de chacun du ou des premiers ensembles consommateurs (5) fonctionne de sorte que la solution de traitement soit fournie par l'intermédiaire du dispositif d'alimentation respectif.

12. Système selon l'une quelconque des revendications 9 à 11, comprenant une pluralité de premiers ensembles consommateurs, et chacun de la pluralité de premiers ensembles consommateurs (5) étant pourvu d'une vanne d'entrée (45) qui est disposée entre le réservoir tampon (37) et le système de conduite haute pression (27), chacune des vannes d'entrée (45) ayant une position fermée et une position ouverte,
l'unité de commande (19) étant reliée à chacune des vannes d'entrée (45) de la pluralité de premiers ensembles consommateurs (5), de sorte à commander la position des vannes d'entrée (45),
l'unité de commande (19) étant en outre conçue de sorte que
dans la première étape, l'unité de commande (19) fasse fonctionner la pompe (21) en mode alimentation, de sorte que l'eau provenant du réservoir central (7) soit fournie à la pluralité de premiers ensembles consommateurs (5), les réservoirs tampons (37) de la pluralité de premiers ensembles consommateurs (5) étant remplis jusqu'à un niveau de remplissage prédéfini,
dans la deuxième étape, le milieu de traitement soit fourni dans les réservoirs tampons (37) de la pluralité de premiers ensembles consommateurs (5) par l'intermédiaire de l'entrée de traitement (48) respective pour le mélanger à l'eau dans le réservoir tampon (37) afin de former une solution de traitement, et
dans la troisième étape, l'unité de commande (19) fasse fonctionner la pompe (21) en mode inversé,
l'unité de commande commandant les vannes d'entrée (45) de la pluralité de premiers ensembles consommateurs de sorte que les vannes d'entrée (45) de ceux-ci soient en position ouverte de sorte que la solution de traitement dans les réservoirs tampons (37) de la pluralité de premiers ensembles consommateurs passe à travers une partie du système de conduite haute pression et le côté amont de la pompe (21).

13. Système selon l'une quelconque des revendications 9 à 12, le raccordement entre le réservoir d'eau central (7) et le côté amont (23) de la pompe (21) comprenant un raccord de remplissage/vidange (15) doté d'une vanne de remplissage/vidange (17) qui a une position ouverte et une position fermée et est reliée fonctionnellement à l'unité de commande (19),
l'unité de commande (19) étant conçue de sorte que, lorsqu'elle fait fonctionner la pompe (21) en mode inversé de sorte que la solution de traitement dans le réservoir tampon (37) du ou des premiers ensembles consommateurs (5) passe à travers une partie du système de conduite haute pression (27) et le côté amont (23) de la pompe (21), l'unité de commande (19) commandant la vanne de remplissage/vidange (17) de sorte
qu'elle soit en position ouverte et la solution de traitement passe à travers le raccord de remplissage/vidange (15) ou
qu'elle soit en position fermée et la solution de traitement soit envoyée dans le réservoir d'eau central (7).

14. Système selon l'une quelconque des revendications 9 à 13, comprenant en outre des seconds ensembles consommateurs (5'), chaque second ensemble consommateur (5') comprenant un dispositif d'alimentation et un réservoir tampon (37), et chaque second ensemble consommateur (5') étant conçu pour fournir du fluide provenant du réservoir tampon (37) par l'intermédiaire du dispositif d'alimentation,
le système de conduite haute pression (27) raccordant le côté aval (25) de la pompe à la pluralité de seconds ensembles consommateurs (5'), le système de conduite (27) étant conçu de sorte que la pompe (21), lorsqu'elle fonctionne en mode alimentation, soit apte à fournir du fluide provenant du côté aval (25) aux seconds ensembles consommateurs (5'), de sorte que le fluide soit reçu dans le réservoir tampon (37) du ou des seconds ensembles consommateurs (5'),
l'unité de commande (19) étant conçue de sorte que
lorsque dans la troisième étape l'unité de commande (19) fait fonctionner la pompe (21) en mode inversé, de sorte que la solution de traitement dans le réservoir tampon (37) du ou des premiers ensembles consommateurs (5) passe à travers une partie du système de conduite haute pression (27) et le côté amont de la pompe (21), la solution de traitement passe dans le réservoir d'eau central (7),
dans une quatrième étape, l'unité de commande (19) fasse fonctionner la pompe (21) en mode alimentation de sorte que la solution de traitement provenant du réservoir central soit fournie au ou aux seconds ensembles consommateurs (5'), la solution de traitement étant reçue dans le réservoir tampon (37) du ou des seconds ensembles consommateurs (5').

15. Aéronef (3), comprenant un système selon l'une quelconque des revendications 9 à 14.
